# EUROPEAN PATENT SPECIFICATION

(11) **EP 0 270 356 B1**
(45) Date of publication and mention of the grant of the patent: **01.06.1994**
(21) Application number: 87310612.4
(22) Date of filing: 02.12.1987
(51) Int. Cl.: C12N 15/82, A01H 1/02, C12N 15/87, C12M 3/00

(54) **Plant-cell transformation by accelerated particles coated with DNA and apparatus therefor.**
Transformation von Pflanzenzellen mittels beschleunigten Teilchen, die mit DNS beschichtet sind und Apparat dafür.
Transformation de cellules de plantes au moyen de particules accélérées couvries avec ADN et l'appareil pour effectuer cette transformation.

(30) Priority: 05.12.1986 US 938570
(43) Date of publication of application: 08.06.1988
(73) Proprietor: AGRACETUS, INC., Middleton, Wisconsin 53562 (US)
(72) Inventor: McCabe, Dennis E., Middleton Wisconsin 53562 (US); Swain, William F., Madison Wisconsin 53705 (US); Martinell, Brian J., Madison Wisconsin 53711 (US)
(74) Representative: Perry, Robert Edward

(56) References cited:
- EP-A- 0 164 575
- EP-A- 0 257 472
- EP-A- 0 267 159
- WO-A-85/01856
- WO-A-86/05516
- Klein et al. (June '85) poster no. 28 at Symposium "Biotechnology in Plant Science": Relevance to agriculture in the eigthies" Ithaca, New York.
- Sanford J.C. (December '88) Tibtech vol. 6 pp.299-302.
- Johnston, S.A. (August '90) Nature vol. 346 pp.776-777.
- Sanford, J.C. et al. (1985) Theor. Appl. Genet. (1985) vol. 69 pp. 571-574.
- Proc. Natl. Acad. Sci. USA, vol. 83, Febr. 1986, pp. 715-719, US; Y.Ohta
- Plant Molecular Biology, vol. 7, 1986, pp. 43-50, M. Nijhoff Publ., Dordrecht, NL; A.C.F. Graves et al.
- Z. Pflanzenphysiol., vol. 94, 1979, pp. 95-99, J. Korohoda et al.
- Nature, vol. 307, Jan. 12, 1984, pp. 108-109, MacMillan Journals Ltd, London, GB; R. Flavell et al.
- Biological Abstracts/RRM, no. 30004894, 1985, pp. 32-33, E. Magnien et al. (Ed.): "Adv. in Agricultural Biotechnology, Genetic Engineering of Plants and Microorganisms Important for Agriculture" , Meeting Copenhagen, Oct. 9-10, 1984, part II5; A. Hepher et al.
- Particulate Science and Technology, vol. 5, no. 1, 1987, pp. 27-37, Hemisphere Publ. Corp.; J.C. Sanford et al.
- Nature, vol. 327, no. 6117, May 7, 1987, pp. 70-73, London, GB; T.M. Klein et al.
- Biotechnology and Ecology of Pollen, Int. Conf. Amherst, July 9-11, 1985, vol. XXIII, pp. 65-70, Springer Verlag, Berlin, DE; I. Negrutiu et al.
- Biological Abstracts/RRM, no. 31-094447, D.L. Mulcahy et al. "Biotechnology and ecology of pollen", Amherst, Mass, US, July 9-11, 1985, pp. 65-70, I. Negrutiu et al.

## Description

The present invention relates to the general field of genetic engineering of plants and relates, in particular to the transformation of exogenous genetic material into the germ line of a plant line by physically introducing the genetic material into pollen of the plants and to apparatus for use in genetic engineering.

There exists much current effort and research being expended toward the genetic transformation of plant species. It is believed that the development of efficient means for transforming foreign genes into plant germ lines will allow the diversity of the genetic stock in commercially important crop species to be widened and to allow functional genes of specific interest to be selectively introduced into crop species. The effort and research to date on the transformation, or genetic engineering, of plant species has achieved results which vary quite dramatically depending on the species of plant.

The principal mechanism which has been used heretofore for the introduction of exogenous genes into plants has begun with the transformation of single plant cells either as protoplasts or in a undifferentiated tissue mass known is a callus. Chimeric genes functional in plant cells have been introduced into single cell plant protoplasts by electroporation and microinjection. However, the most widely used transformation technique used to date has taken advantage of a natural trait of the plant pathogen Agrobacterium tumefaciens, which has the innate ability to transfer a portion of the DNA from a Ti (Tumor-inducing) plasmid harbored in it into an infected plant cell. By inserting foreign genes into plasmids in Agrobacterium which carry certain sequences from the Ti plasmid, the bacterial transformational trait can be used to transport the foreign genes into the genome of the infected plant cells. Agrobacterium-mediated plant cell transformation has been found to work reasonably well in many model crop species, such as tobacco, petunia and carrot, but does suffer from two significant limitations. The first limitation is that the mediation can only be done on an individual cellular level, typically with somatic tissues, which then must be regenerated artifically into a whole plant. This limits the applicability of Agrobacterium-mediated genetic transformation to those crop species which can readily be regenerated from types of tissues which are susceptible to Agrobacterium infection. A second limitation is that the natural host range of Agrobacterium includes only dicotyledonous plants and a limited number of monocot species of the Liliaceae family. Therefore Agrobacterium-mediated transformation has not been proven to be an effective tool for monocot species of commercial interests, such as the cereal crop species. Another difficulty with Agrobacterium-mediated transformations is the generation of somoclonal variants, which spontaneously arise in plant tissues in tissue culture, which may complicate identification of transformants.

It has been demonstrated that at least some chimeric gene constructions are effective for expression of foreign genes in most plant cells. The functionality of these chimeric constructions in monocots as well as dicots has been demonstrated by the transformation of maize protoplasts in culture through such techniques as electroporation. However, no currently known methodology exists to regenerate whole maize plants, or whole plants of any other important crop species, from such protoplasts. No whole, intact transformed maize plants, for example, are known to have been generated. Nevertheless genetic transformation of lines of maize and other crop species is a desired objective because of the great agricultural value of the common crop plants and the potential to improve their value and productivity.

There has been at least one suggestion previously that maize plants can be genetically transformed by genetic transformation of their pollen. Published PCT patent application WO 85/01856 to DeWet purportedly describes a method for the transfer of exogenous genes into flowering plants by transforming the pollen of the plants. Attempts by others to transform the pollen of plants and regenerate transformed plants have failed. Sanford et al., Theor. Appl. Genet., 69(5-6), 571-74 (1985). A report of one similar result has been made. Ohta, Proc. Natl. Acad. Sci. USA, 83:715-719 (1986).

A method of particle-mediated transformation of onion epidermal cells was disclosed by Sanford and Klein at a symposium entitled "Biotechnology in Plant Science": Relevance to Agriculture in the Eighties", Ithaca, NY, USA, 23-27 July 1985, (poster number 28). The disclosed method uses a ballistic device to accelerate DNA-bearing tungsten spheres into the cells. Further reports of this work were published after the priority date of the present application, in Nature, 1987, vol 327, pages 70-73, and Particulate Science and Technology, 1987, vol 5, pages 27-37.

The present invention provides a method of making genetically transformed living cells comprising the steps of: preparing copies of a foreign gene including a coding region and flanking regulatory sequences effective to express the coding region in the cells; coating copies of the foreign gene onto biologically inert carrier particles; layering the particles on a planar carrier sheet, accelerating the carrier sheet by impacting the carrier sheet with a shock wave, and restraining the carrier sheet such that the momentum of the carrier particles carries them from the sheet, so that some particles lodge in the interior of some of the cells; and screening for transformed cells.

In a particular embodiment of this method, the invention relates to a method of genetically transforming a plant line which comprises the steps of: preparing a DNA sequence including a foreign gene and regulatory sequences; coating the DNA sequence onto biologically inert particles; physically accelerating the particles carrying the DNA at pollen of the plant to lodge the particles in the pollen; pollinating a female parent plant with the pollen; and selecting from progeny of the pollination for transformed plants.

The present invention is also directed towards maize pollen comprising in it a foreign chimeric gene and at least one carrier particle which physically carried the foreign gene into the pollen.

The present invention is further directed toward the genetic transformation of not only maize but other important crop plants through pollen transformation and without the need for tissue culture or regeneration of plants.

The present invention further provides apparatus for injecting carrier particles carrying DNA into living cells on a target, the apparatus comprising:
a planar carrier sheet; arranged to receive on the surface of the sheets a layer of biologically inert carrier particles coated with DNA;
means for generating a shock wave sufficient to accelerate the carrier sheet with the carrier particles thereon to travel toward the target; and
restraining means disposed in the path of travel of the carrier sheet towards the target and arranged and constructed so as to restrain the carrier sheet when the carrier sheet is accelerated by a shock wave so that carrier particles are accelerated into cells on the target.

It is an advantage of the present invention in that the method is relatively quick and efficient, readily verifiable, and replicable.

It is an advantage of the method of the present invention and the materials produced therefrom that foreign genetic material, characterised or uncharacterised, can readily and rapidly be introduced into any desired genetic background of maize for crop breeding, molecular biology, or other similar agronomic or scientific purposes.

An additional advantage of the method of the present invention is that numerous, i.e. thousands, of transformant events are possible and feasible because of the ease of performing the process in contrast to prior somatic-cell transformation techniques or micro-injection which are difficult to perform or require cell-by-cell treatment.

Other objects, advantages and features of the present invention will become apparent from the following specification when taken in conjunction with the accompanying drawings.

Fig 1 is an exploded perspective view of an apparatus constructed to perform the method of the present invention.

Fig. 2 is a schematic illustration of the plasmid manipulations in the process of making plasmid pCMC 1208.

Fig. 3 is a schematic illustration of the plasmid manipulations in the process of making the plasmid pCMC 1022.

In the practice of pollen-mediated plant genetic transformation conducted in accordance with the present invention, DNA is physically delivered into the cytosol of plant pollen, the DNA being carried on individual small particles of biologically inert material which are accelerated at the pollen so that the particles enter the individual pollen cells but neither destroy nor incapacitate them. It has been found that DNA delivered in such a fashion will be incorporated into the genetic material of the progeny of this pollen. Thus transforming pollen in this fashion allows for the genetic engineering of plants and plant lines.

There are several factors which influence successful pollen-mediated transformations. The manner in which the particles are accelerated is preferably carefully arranged so that the individual DNA-bearing particles have a proper momentum and velocity, and the particles themselves are in a relatively uniform pattern, when contacting the pollen that they penetrate a significant number of pollen cells without biologically disabling them. Furthermore, the DNA on the particles should be stable and capable of transforming plant cells and expressing the desirable trait in the plant cells. In addition, the DNA itself may contain a selectable marker which can be detected in putatively transformed plant seeds or plantlets in order to verify the specific plants in which genetic transformation has occurred. If the transformation frequency is high enough, such a selectable marker may not be necessary.

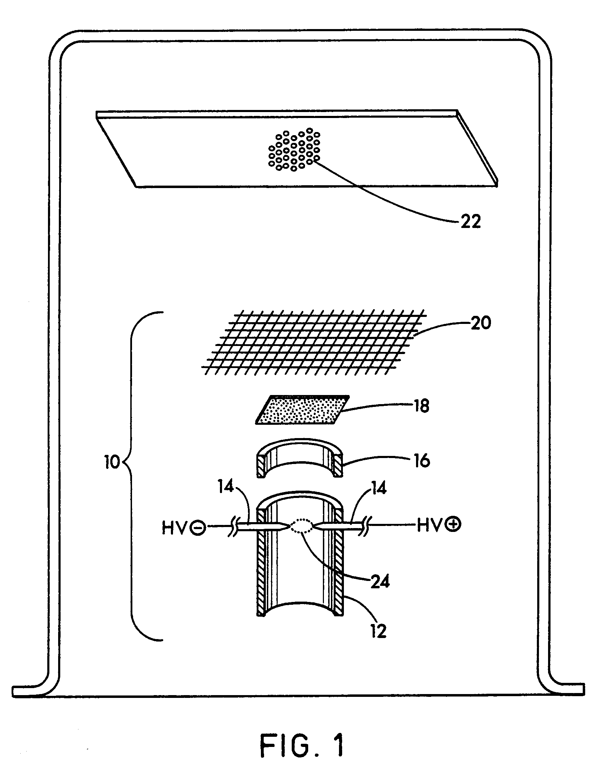

There are many types of mechanical systems which can be envisioned to accelerate biologically inert small carrier particles. Possible mechanisms might include ballistic explosive acceleration of particles, centrifugal acceleration of particles, electrostatic acceleration of particles or any other analogous system capable of providing momentum and velocity to small inert particles. One novel method preferred by the applicants here is illustrated in schematic fashion in Fig. 1. The method illustrated here makes use of a shock wave created by high voltage electrical discharge. In Fig. 1, and generally indicated at 10, is an accelerator for accelerating the inert particles using this method. Also shown in Fig. 1, and generally indicated at 22, is a target surface for carrying the pollen target thereon.

The accelerator 10 consists of several parts. A spark discharge chamber 12 has provided extending into its interior a pair of electrodes 14. The geometry of the spark discharge chamber 12 is not believed to be critical to the present invention as long as the chamber is configured to develop and present a shock wave of proper character and proper direction that it can be used to propel the carrier particles. The applicants have found that a section of 13 millimeter interior diameter polyvinyl chloride plastic pipe is satisfactory for use as the spark discharge section 12. The electrodes 14 are extending oppositely into the interior mounted approximately 5 millimeters below the top of the spark chamber 12. The electrodes 14 themselves are formed by threaded bolts extending into suitable threads formed in the interior sidewall surfaces of the spark chamber 12 wall itself. The ends of the threaded bolts forming the electrodes 14 are protected with an arc resistant alloy obtained from high electric voltage relay contact points cut to a size of approximately 2 millimeters by 2 millimeters by 3 millimeters and soldered to the ends of the threaded bolts. The gap between the electrodes 14 can be adjusted by appropriately threading the bolts into or out of the spark chamber 12. The preferred gap for discharge voltage of approximately 15 kilovolts between the ends of the electrodes is between 1 and 1.5 millimeters. The method of fabricating and mounting the electrodes 14 themselves is clearly subject to wide variation, although it is preferred that the electrodes be highly durable and that the distance of the spark gap between the electrodes be readily adjustable.

A spacer ring 16 is provided above the spark chamber 12. The spacer ring 16 may be constructed out of the same PVC pipe as the spark chamber 12 itself and preferably be cut to a vertical length of 6 millimeters. In a fixed apparatus for transformations of a single crop species, the spacer ring 16 could be constructed merely as a vertical extension of the spark discharge chamber 12, although a removable and replacable spacer ring 16 allows adjustment of the distance from spark discharge to carrier sheet to be varied so that the force of particle acceleration can be varied by conditions or by species. The spacer ring 16 may be left open at the top if a large carrier sheet 18 is used, but may also advantageously have its top opening partially restricted by a suitable closure to form a rectangular opening approximately 9 by 13 millimeters. Placed atop the spacer 12 is a carrier sheet 18. The carrier sheet 18 is a planar, light sheet formed of suitable size to be placed resting atop the spacer ring 16. The carrier sheet 18 is formed of flexible biologically inert sheet material capable of carrying biologically inert small particles thereon. The carrier sheet 18 functions to transfer the force of a shock wave from a spark discharge into acceleration of the carrier particles. It has been found that the carrier sheet 18 may advantageously be formed from 1 mil or 0.5 mil plastic coated aluminized mylar, with the 0.5 mil sheets being preferred since in practice they result in better penetration into the pollen. As a general practice the smaller the actual surface area of the carrier sheet 18, the better penetration is obtained by the carrier particles into the pollen. This consideration regarding penetration is balanced by the need to have the carrier sheet of a size which is easy to handle and which provides an impact pattern over a large enough field to be able to impact large numbers of pollen cells in each individual injection. A carrier sheet size of 9 by 11 millimeters has been found to provide a good size yielding good penetration in a desirable impact pattern of the particles onto the pollen target.

The carrier sheet also functions to arrange the pattern of the particles as they contact the target surface. An even uniform pattern of particles is highly desirable to ensure that as many cells on the target as possible are impacted, in order to maximize the yield of transformants. Non-transformed cells, pollen or otherwise, may be at a competitive advantage with transformants or may be partially debilitated by the carrier particles. Therefore it is desirous to reach as close to 100 percent injection into the target cells as is possible, and a uniform layer and pattern of particles on a carrier sheet 18 aids this objective.

As to the carrier particles themselves, any high density material which is biologically inert should be acceptable for use as the DNA carrier particles within the context of the present invention. Metallic materials are preferred, such as tungsten and gold, which have a density of 19. Iridium might also be preferable, having a density value of 22, but has not been used by the applicants because it is only easily available in a relatively course powder, whereas spherical particles are preferred. Tungsten is also probably less desirable compared to gold because it tends to oxidize in air in the presence of even trace moisture. Such an oxidation layer on the carrier particles tends to bind the particles together causing severe increase in average particle size as the particles aggregate together. Particles which are clumped in irregular aggregations are less desirable for the practice of the present invention since such aggregations will vary widely in their mass and size, thus leading to difficulty in obtaining regularly replicable results. It has been found that gold is an optimal material for the particles within the present invention since it has high density, is relatively inert to both biological materials and to oxidation, and is readily commercially available in the form of spheres having a diameter of 1 to 3 micrometers. Suitable DNA sequences may be applied to the gold particles and the gold particles may be applied to the carrier sheet in a manner which will be discussed in further detail below.

Located above the carrier sheet 18 is a retainer screen 20. The retainer screen 20 is a 100 mesh stainless steel screen physically mounted in a plastic holder approximately 5 millimeters above the top of the spacer ring 16. The retainer screen 20 functions to restrain the carrier sheet 18 so that it does not proceed to the target.

The target surface 22 is a planar sheet of material capable of suspending the target cells, i.e. pollen or other plant cells, thereon. In practice it has been found that an easily useable target is a petri dish 60 millimeters by 15 millimeters inverted over the top of the assembly holding the retainer screen. Spacing from the retaining screen 20 to the target cells on the target surface 22 is therefore preferably approximately 15 millimeters. Spacing greater than 15 millimeters, under the conditions of voltage and atmospheric pressure described below, leads to reduced penetration of carrier particles into the pollen while a spacing of less than 10 millimeters results in crushed cells in the event that the retaining screen 20 deforms under the force of the blast.

If pollen is used as the target cells, the pollen must be applied to the target in such a fashion that the target may be inverted with the pollen remaining viable. Since pollen in general is sensitive to moisture, the method used to adhere the pollen to the target should be as moisture-free as possible. It has been found that mineral oil is useful as such an adhesive. If a thin layer of mineral oil is applied to the bottom of a Petri dish to be used as the target surface 22, pollen is dusted into the dish, and then the dish overturned to remove excess pollen, it has been found that a relatively uniform monolayer of pollen grains remains on the target which will remain in place during particle injection and which remains viable. If cells other than pollen are used in this apparatus other support media, such as agar, may be more appropriate.

The entire assembly of the particle accelerator 10 and the target surface 22 must be partially evacuated so as to prevent the force of atmospheric drag from slowing the particles and/or the carrier sheet 18. The vacuum should be only a partial vacuum since a high vacuum would desiccate the target pollen cells, rendering them non-viable. A vacuum of 460 to 480 millimeters of mercury has been found sufficient and advantageous.

In the simplest explanation of the operation apparatus of Fig. 1, the process of firing the accelerator 10 begins with the placement of a drop 24 of distilled or demineralized water between the electrodes 14. The amount of water must be selected so as not to dampen the arc which will occur between the electrodes but yet be of sufficient volume to create a shock wave in the interior of the spark chamber 12 when the discharge does occur. The preferred volume of water has been found to be approximately 2-4 microliters. This amount of water may be applied by pipette suspended between the ends of the electrodes 14. The water droplet 24 will bridge the gap between the electrodes and remain in place.

The spacer ring 16 is then placed upon the top of the spark chamber 12 and the carrier sheet 18 is placed on the top of the spacer ring 16. The retaining screen 20 is mounted in place 5 millimeters above the carrier sheet 18 and the target surface 22 consisting of the overturned Petri dish is placed above the mounting of the retaining screen 20. The assembly is then evacuated to 480 millimeters of mercury.

External to the apparatus illustrated in Fig. 1, a voltage supply is connected to generate 15,000 volts DC. The 15,000 volts DC is then applied to a 1 microfarad capacitor, which is then disconnected from the voltage source. By throwing a suitable switch, the 15,000 volt charge on the capacitor is then applied between the electrodes 14.

When the voltage is applied, an electric discharge arc jumps between the two electrodes 14. The arc instantly vaporizes the small water drop extending between the electrodes. A shock wave from the explosive vaporization of the water drop propagates throughout the interior of the spark chamber 12. When the shock wave reaches the carrier sheet 18, the carrier sheet 18 is lifted vertically off the spacer ring 16 and is accelerated toward the retaining screen 20. When the carrier sheet 18 hits the retaining screen 20, the carrier sheet 18 is restrained in place and the particles carried on the carrier sheet 18 leave the carrier sheet and fly freely across the distance to the cells resting on the target surface 22. If the apparatus has been properly constructed and adjusted, and the procedure properly followed, a significant percentage of the carrier particles will arrive at the target with a correct velocity to penetrate the cells carried on the target surface 22, without destroying an unacceptable percentage of the cells. The cells on the target surface 22 may then be removed from the target surface 22 and selected as appropriate to segregate transformants from non-transformants. If pollen is used, as preferred, in the process, the pollen is then removed from the target surface 22 and hand pollinated onto fertile female flowers, such as maize silks, which will then set seed, or kernels. The seed can be harvested, planted and evaluated for the morphological or biochemical traits conditioned by the DNA carried on the carrier particles into the pollen. Alternatively, immature embryos may be excised from the developing seed tissues and the embryos grown out in appropriate tissue culture into small plantlets or into whole plants. The plants or plantlets, or tissues from them, can then be tested for selection on the basis of a selectable marker carried in the DNA transformed into the pollen cells. Suitable selectable markers would include exogenous resistance traits, such as herbicide or antibiotic resistance, or dominant morphological traits whose expression can be observed.

It is to be understood that while the apparatus of Fig. 1 has been specifically developed for the process of pollen mediated plant transformation in accordance with the present invention, the apparatus itself is also useful for accelerated particle transformation of other tissue types, plant, animal or bacterial, as well. The apparatus allows for easy adjustment of the particle force by varying the spacing or the discharge voltage. It is relatively simple to operate, efficient and stable so that results may be replicated.

Within the preferred process of the present invention, the process for applying the DNA sequences to the particles, the process for layering the particles into the carrier sheet, and the process for preparing the DNA for plant transformation all may require particular attention. Each of these details of the preferred process will be discussed in turn, in relation to transformation of plants

The DNA sequence including a foreign gene prepared in the form suitable for plant transformation can be simply dried onto naked gold or tungsten pellets. However, DNA molecules in such a form tend to have a relatively short period of stability and tend to degrade rather rapidly due to chemical reactions with the metallic or oxidate substrate of the particle itself. It has been found, by contrast, that if the carrier particles are first coated with an encapsulating agent the DNA strands have greatly improved stability and do not degrade significantly even over a time period of several weeks. A suitable encapsulating agent has been found to be polylysine (molecular weight 200,000) which can be applied to the carrier particles before the DNA molecules are applied. Other encapsulating agents, polymeric or otherwise, are also believed useful as similar encapsulating agents. The polylysine is applied to the particles by rinsing the gold particles in a solution of 0.02% polylysine and then air drying or heat drying the particles thus coated. Once the metallic particles coated with polylysine have been properly dried, DNA strands can then be loaded onto the particles. The DNA may be loaded onto the particles at a rate of between 3 and 30 micrograms of DNA per milligram of gold bead spheres. The practice has been to add to 100 micrograms of DNA and 30 milligrams of 1-3 micron gold spheres precoated with polylysine, sequentially 5 microliters of 10 mM Na₂HPO₄ and then 5 microliters of 10 mM CaCl₂ to provide a fine CaHPO₄ precipitate which forms as the solution dries. The precipitate carries the DNA with it onto the beads. Once the beads and the phosphate and calcium chloride solution have been mixed with the DNA, the suspension is dried under a nitrogen (N₂) stream with frequent stirring. Once dried the precipitate is immediately resuspended in 100% ethanol for the process of placing the particles onto the carrier sheet.

In applying the particles to the carrier sheet, it is preferred for the successful operation of this procedure to form a uniform and reproducible layer of the carrier particles on the carrier sheet. To do this, the particles cannot be simply dusted onto the carrier sheet, since they tend to aggregate and are thus distributed unevenly in a non-reproducible fashion on the sheet. In particular, moisture or water content on the sheet will disrupt the application of the particles to the sheet and result in undesirable aggregations. Therefore, it is first necessary to precoat the mylar sheet with a hydrophilic coating intended to prevent water spotting when applying the carrier particles. It has been found that hydroxy ethyl cellulose works well for this purpose, although other similar treatments, such as acid hydrolyzed cellulose, are also feasible. A solution of 1% hydroxy ethyl cellulose is wiped over the plastic coated aluminized mylar, which is then rinsed with ionized water and air dried. The carrier particles, with the precipitated coating containing the DNA strands, suspended in 100% ethanol, is then applied to the carrier sheet. It has been found that 50 or 100 microliters of a well stirred suspension of the ethanol with the carrier particles can be successfully pipetted onto the mylar sheet in a reasonably uniform and reproducible fashion. The pipetted aliquot of this suspension is then allowed to settle in a closed petri dish for at least 30 seconds. The petri dish must be closed to prevent eddy currents from forming from room air currents and from a high rate of evaporation, such eddy currents potentially causing excessive drifting of the particles and therefore a non-uniform distribution of particles on the sheet. After the settling period, the meniscus is broken and the excess ethanol is drained away. The residual ethanol is removed by evaporation in a partially opened petri dish.

This process is intended to place the carrier particles coated with the precipitate containing DNA strands on the mylar carrier sheet. A good median rate which is found successful within the present invention is approximately 0.1 milligram of carrier particles carrying the precipitate and DNA applied to a 9 by 11 millimeter area of the carrier sheet. Such a density of carrier particle application to the carrier sheet gets good survival of pollen and also a high penetration of pollen grains by the accelerated particles. The actual acceleration and penetration of the grains by the particles will vary both with the pollen size and diameter, and the number of carrier particles can obviously be varied to give more or fewer particles per cross-sectional area of the target cells as desired.

The DNA for use within the present invention must be constructed in a vector appropriate for expression of the exogenous gene in the cells of maize, or whatever other plant is being utilized within the present invention. The DNA sequence can be chimeric, but full intact non-chimeric genes from other plants species or lines of the same species may also be used. Vectors suitable for expression in plants generally must include, besides the coding sequence of the desired exogenous gene, appropriate flanking regulatory sequences such as a suitable promotor capable of promoting transcription and expression in vivo in plant cells and a translation terminator capable of signalling the end of transcription or the appropriate processing of the RNA in such a fashion that will allow suitable translation of messenger to produce protein synthesis. It has been previously demonstrated that plant gene promoters capable of causing coding sequence transcription and expression in dicot plant cells are also effective in monocots, such as corn, on a cellular level although with lowered efficiency in some cases. Fromm et al., Proc. Natl. Acad. Sci. USA, 82:5824-5828, September 1985. Such promoters include the nopaline synthase promoter from the plant pathogen Agrobacterium tumefaciens and the CaMv 35S promoter derived from the cauliflower mosaic virus sequence. A suitable termination sequence effective in plants is the polyadenylation sequence from the nopaline synthase gene of Agrobacterium tumefaciens. The plant expression vector may also contain a selectable marker operative in plant cells to allow for selection of transformant plants. The selectable marker may condition a trait which may be assayed biochemically or a phenotypic trait which may be observed in the progeny plant. Clearly if a non-chimeric intact gene, with flanking regulatory sequences, from the same or another plant is used in the present process, chimeric promoter or control sequences are unnecessary and the gene may be used with its native sequence.

While the process of the present invention has been described in particular detail with regard to pollen-mediated transformation of maize, it should be understood that there is nothing intrinsic to the process that is of necessity limited to maize, and the process is equally suitable for pollen transformation of other cereal crops as well as dicot crops such as soybean and cotton, and most other plants as well. The procedure for handling pollen of other species may need to be varied and the spacing of the parts of the apparatus critical to carrier particle velocity may need to be varied depending on the species, but the basic apparatus and procedure may be used in other plant species.

In addition, while the process of the present invention is directed toward pollen-mediated plant transformation, the apparatus disclosed herein is equally suitable for use in transformation of other plant tissues, such as embryogenic callus or somatic embryos, or any other plant or other tissue in culture.

Since not all of the pollen will have carrier particles inserted into them, and since not all pollen cells or progeny zygotes will uptake the DNA into their genome, it will be necessary to screen the progeny plants at some stage to select for transformants. If it is desired to transform a given foreign gene into a plant, the gene may be inserted into a chimeric expression vector. The chimeric expression vector could then be transformed into plant cells along with a selectable marker plasmid, such as pCMC 1022 described herein below. The two vectors (foreign gene and selectable marker) can be ligated together to make one plasmid, or the two vectors can be cloned separately and then applied together to the same carrier particles. In either event, the progeny produced are screened for the marker to select transformed progeny. While the use of such a selectable marker may be desirable in some circumstances, it may be omitted if a suitable morphological or biochemical test exists to screen for the transformed progeny. A morphological screening test could be for a dominant phenotypic trait in the progeny. A suitable biochemical screening test could be a so-called "Southern" blot hybridizing probe for the existence of the transforming DNA itself in the genome of the progeny plants.

### EXAMPLES

### 1. Construction of Vectors

### A. Antibiotic Resistance.

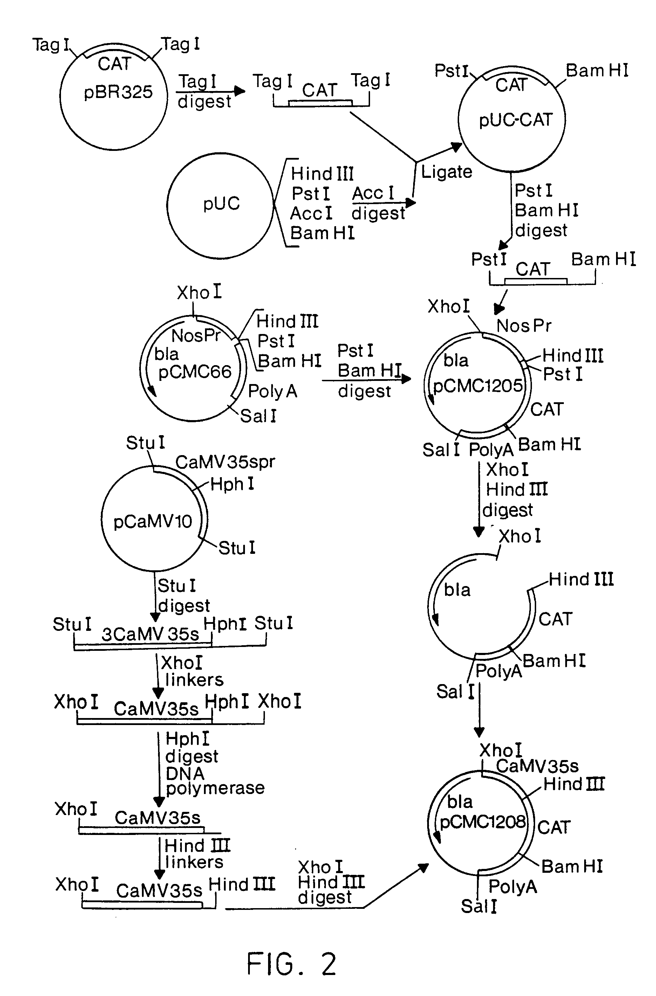

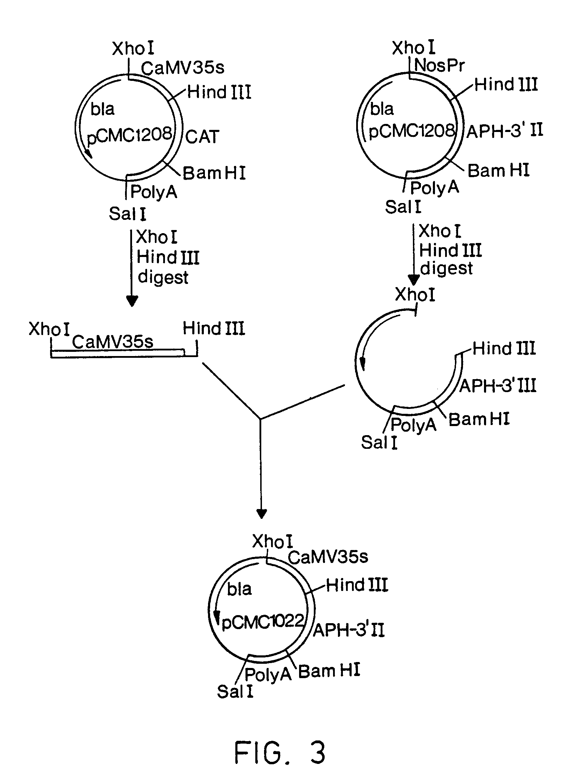

The construction of suitable plant expression vectors is illustrated in schematic fashion in Figs. 2 and 3. Fig. 2 illustrates, in schematic form, the construction of a plant expression vector pCMC 1208. The construction of the plasmid pCMC 1208 began with the digestion of the plasmid pBR 325 (Bolivar, F. Gene 4:121-136 (1978)) with the restriction endonuclease Taq I. The plasmid pBR 325 contains a coding sequence for the antibiotic resistance gene chloramphenicol acetyl transferase (CAT) which is exised from the remainder of the plasmid by Taq I digestion. After digestion of pBR 325, the fragments were resolved by electrophoresis in an agarose gel and the fragment containing the CAT gene was excised. The CAT fragment was then ligated into the plasmid pUC 9 (Viera & Messing, Gene, 19:259-268 (1982)) which had previously been digested with the restriction enzyme Acc I. The fragment ends produced by Taq I and Acc I are complementary in this case and thus the strands were directly ligatable. The resulting plasmid, designated pUC-CAT in Fig. 2, contained the CAT coding sequence flanked by portions of the polylinker from pUC 9. This plasmid was digested with Pst I and Bam HI, and the smaller of the two fragments was isolated by gel electrophoresis. This fragment was then ligated to an intermediate plant expression vector pCMC 66, which had been previously digested with Pst I and Bam HI, to form the CAT expression plasmid pCMC 1205. The plasmid pCMC 66 contains the nopaline synthase promoter (Nos Pr) from Agrobacterium tumefaciens and a nopaline synthase polyadenylation sequence (Poly A), from the same organism, surrounding six plasmid unique restriction sites. The plasmid pCMC 66 also carries a version of the beta-lactamase gene (bla) which expresses resistance to the antibiotic ampicillin in bacteria, so that ampicillin resistance can be used as a selection marker in subsequent recombinations performed in E. Coli.

The plasmid pCaMV 10 (Gardner et al., Nucl. Acids Res 9:2871-2888(1981)) was digested with Stu I and the fragment containing the cauliflower mosaic virus 35 promoter (CaMv 35s) was joined to synthetic Xho I oligonucleotide linkers. The fragment was then digested with Hph I, treated with a DNA polymerase to generate blunt ends, and then joined to synthetic Hind III oligonucleotide linkers. Digestion of this fragment with both Xho I and Hind III produced a fragment containing the CaMv35s promoter and transcription start site modified at its ends by the addition of the restriction site sequences.

The nopaline synthase promoter was excised from pCMC 1205 by digestion of the plasmid with Xho I and Hind III. The larger of the two fragments thus produced was ligated with the CaMv35s promoter fragment to produce pCMC 1208, a plant expression vector having the CaMv35s promoter, the CAT coding sequence and the nopaline synthase polyadenylation sequence in order. The CaMv35s promoter and poly A sequences served as the flanking regulatory sequences for the CAT coding sequence.

Both of the plasmids pCMC 1205 and pCMC 1208 were tested for activity in maize by electroporation into protoplasts, followed by an assay for CAT activity. Both constructions proved active in maize cells, but pCMC 1208 proved significantly higher in level of activity, and thus was selected for plant transformation experiments.

The plasmid pCMC 1208 was used for the pollen-mediated genetic transformation of maize in the apparatus and process of the present invention. However, it was found that the assay for CAT activity had a high background level in maize tissue and thus the CAT gene was considered not an optimal marker in maize. Accordingly, the plasmid was further manipulated to insert another antibiotic resistance gene, of more selectivity in maize, in the vector in place of the CAT gene, as illustrated in Fig. 3.

The plasmid pCMC 1021 contains the nopaline synthase promoter and the nopaline synthase polyadenylation sequence flanking a coding region for the enzyme aminoglycoside-3-phosphotransferase II (APH 3ʹII) which conditions for resistance to aminoglycoside antibiotics such as kanamycin. Since electroporation experiments revealed the CaMv35s promoter to be much more effective in maize than the Nos Pr, it was decided to transfer the CaMv35s promoter to pCMC 1021. The CaMv35s fragment from pCMC 1208, as illustrated in Fig. 3, was isolated by digestion with Xho I and Hind III and isolation by electrophoresis. The plasmid pCMC 1021 was also digested with Xho I and Hind III and the larger fragment isolated and ligated with the CaMv 35s fragment to produce pCMC 1022. In plasmid pCMC 1022 the coding sequence from APH3ʹ II is flanked by the regulatory CaMV35s and Nos pA sequences.

The plasmids pCMC 1208 and pCMC 1022 were both demonstrated to be effective for transformation and expression in individual cells of tobacco, cotton, soybean and corn through electroporation transformation and protein assays. Plant cells transformed in culture with the APH 3ʹ II have been demonstrated to be resistant to kanamycin for cotton, soybean and corn cells.

### B. Endosperm color marker.

A plasmid referred to as pMBzRI was obtained which contains an approximately 9.9 kilobase Eco RI fragment of the maize genomic DNA which includes the entire gene encoding the enzyme UDP glucose-flavored glucosyl transferase, an enzyme which is required for the synthesis of anthocyanin pigments in corn. The genomic fragment contains extensive both 5ʹ and 3ʹ flanking DNA and thus is expected to include appropriate regulatory sequences effective in maize to express the gene. Since the cloned gene is a full-length copy of the normal, functional maize gene, it would be expected that the cloned gene would be fully active and function appropriately in maize cells.

The enzyme itself, UDP glucose-flavonol glucosyl transferase, is useful as a selectable marker for genetic transformation in maize because maize lines are available which carry recessive mutations which inactivate the endogenous gene. Since the enzyme is non-essential for plant growth and development, the plants of the mutant lines are normal except for the lack of the red anthocyanin pigments produced in various tissues of wild-type or non-mutant maize plants. Introduction of the wild-type gene into homozygous mutant lines results in the production of the enzyme and thus ultimately the production of anthocyanins, so that transformant plants can be easily identified due to their characteristic color. Thus the plasmid pMBzRI is suitable for use, without modification, as a model expression vector in maize and, when coupled to another gene of interest, as a conveniently screenable transformation marker. This is an example of a potentially useful non-chimeric gene.

### 2. Transformation of Maize using pCMC 1022

A quantity of 1-3µm gold spherical beads for use as carrier particles were pre-coated with polylysine by being rinsed in 0.02% polylysine and air drying. 100 micrograms of pCMC 1022 DNA in aqueous solution had added to it 33 mg coated beads, and then sequentially 5µl of 10 mM Na₂HPO₄ and 5 µl of 10 mM CaCl₂ which formed a fine precipitate as the solution was dried in a N₂ stream. The dried precipitate-coated beads were then re-suspended in 100% ethanol and deposited onto 2.0 mil plastic coated aluminized mylar sheets approximately 1 cm by 1 cm. The coated beads were applied to give a final density of 0.1 mg/cm² on the mylar carrier sheet.

The carrier sheet carrying the coated beads thereon was mounted atop the spacer 16 in the apparatus of Fig. 1. Pollen was gathered by hand from Early Sun-Glo sweet corn. The bottom of a 60 mm Petri dish was lightly coated with mineral oil and pollen was dusted onto it. Excess pollen was removed by inverting the Petri dish to leave a monolayer. The Petri dish was used as the target surface 22 in the apparatus of Fig. 1.

A vacuum of 55-60 mm of Hg was applied to the assembled apparatus of Fig. 1. A 15 KV discharge from the 1µF capacitor was discharged through the electrodes 14 accelerating the coated particles at the pollen on the target surface 22.

The process of preparing beads and pollen and firing the apparatus of Fig. 1 was repeated several times until an adequate supply of treated pollen was accumulated. The treated pollen was brushed off the bottom of the Petri dish with a brush and hand pollinated onto the silks of female plants of Kaltenberg 390 and CFS 5504 hybrids. The silks were physically segregated from other pollen.

From the ears pollinated in this fashion, 52 kernels were produced. The immature embryos were excised from the ears 14 days after pollination and placed in culture on a corn embryo tissue culture medium containing 50 parts per million Kanamycin. The seedlings which grew up on the medium were assayed directly for APH3ʹ II activity and three seedlings assayed positive, indicating the APH3ʹ II enzyme was being expressed in the tissues of the seedling thus indicating successful transformation of these plants.

One of these plants were placed on a nonselective medium before transfer to a greenhouse for further growth. Leaf tissue was analyzed for continued APH 3ʹ II activity which was positive.

The presence of pCMC 1022 sequences in the DNA isolated from this plants and from one plant which did not assay positive was demonstrated by the Southern hybridization-technique. Southern, J. Mol. Bio., 98:503-577 (1975). DNA was isolated from control and test corn leaf samples by micromodification of the cetyl-trimethylammonium bromide procedure of Taylor and Powell, Focus, 4:4-6 (1982). 10 µg of each DNA sample was digested with the restriction enzymes Ava I and Hind III, resolved by electrophoresis in an agarose gel, transferred to a nylon membrane, and hybridized with a ³²P-labeled probe corresponding to the non-coding strand of the APH II coding region. After washing the filter, hybridizing DNA fragments were visualized by auto radiography.

The expected 1kb fragment was not found in either plant. However, each of the plants exhibited an approximately 4kb fragment which hybridized with the APH-3ʹ II probe and which was not found in any of the control non-transformed control samples of maize DNA. One of the two plants (the one positive for APH II) also exhibited a 3.7 kb specifically hybridizing fragment. The fact that the observed fragment is not the expected size is not too surprising since complex restriction patterns are generally observed for DNA transfected into plant and animal cells. Perucho et al., Cell, 22:309-317 (1980); Kiens et al., Plant Mol. Biol., 5:223-224 (1985); Paszkowski et al., EMBO J., 3:2717-2722 (1984); Riggs and Bates Proc. Natl. Acad. Sci. USA, 83:5602-5606 (1986). Furthermore, in eucaryotic cells DNA can be modified, e.g. by methylation, in ways that alter its expected restriction digestion pattern. Chandler and Walbot, Proc. Natl. Acad. Sci. USA, 83:1767-1771 (1986). Both Ava I and Hind III, the restriction endonucleases used in this example, are known to be inhibited by specific methylation within their recognition sequences. McClelland and Nelson, Nucleic Acids Res, 13:r201-r207 (1985). The 4 kb fragment length is equivalent to the plasmid unit length of the pCMC 1022 plasmid and suggests that these plants contain tandemly duplicated copies of the plasmid. Digestion with either enzyme alone would then produce the plasmid-length fragments observed. The 3.7 kb fragment appears to result from a rearrangement of the plasmid, perhaps at its juncture with indigenous maize DNA.

Two additional replicates were done utilizing the identical procedure as described above with pollen from CFS 5504 plants placed on silks of CFS 5504 plants. Two plants generated from the procedure were selected at random from the plants produced, and were assayed for APH 3ʹ II and were analyzed by Southern blot. Both plants failed to show APH 3ʹ II activity but evidenced the 4.0 kb hybridizing fragment in their genome.

Another replicate using identical procedues with pollen from A188 and a Flint maternal plant again resulted in progeny from which a plant was selected at random for analysis. The leaves of this plant tested positive for APH 3ʹ II and also evidenced the 4.0 kb fragment in the Southern blot analysis.

### 3. Use of pCMC 1022 with Other Genes

To transform other genes of interest into maize or other plants, plasmid pCMC 1022 may be used in any of several ways. The APH3ʹ II coding sequence can be deleted by digestion of pCMC 1022 with Hind III and Bam HI and another gene sequence of interest prepared with appropriate ends can be ligated in its place. If the gene of interest can reasonably be selected for, the plasmid may then be directly used for transformations. If the gene of interest is separately prepared with appropriate regulatory sequences, and a selectable marker is desired, the gene of interest with its regulatory sequences can be inserted in any of the sites in the polylinker upstream of the CAMv35s sequence in pCMC 1022. Another alternative to make use of the pCMC 1022 selectable marker is to prepare the gene of interest, in pCMC 1022 or in any other plant expression vector, and to coat pCMC 1022 and the gene expression vector together onto carrier particles as disclosed herein for transformation into plant cells.

The plasmid pCMC 1022 was deposited with the American Type Culture Collection, 12301 Parklawn Drive, Rockville, MD, USA, on November 14, 1986 under ATCC accession No. 67269.

The above deposit was made pursuant to a contract between the ATCC and the Cetus Corporation, a partner in the assignee of the present invention. The contract with the ATCC provides for permanent availability of the progeny of these cell lines to the public on the issuance of the US patent describing and identifying the deposit or the publication or laying open to the public of any US or foreign patent application, whichever comes first, and for availability of the progeny of these cell lines to one determined by the U.S. Commissioner of Patents and Trademarks to be entitled thereto according to 35 USC Section 122 and the Commissioner's rules pursuant thereto (including 37 CFR Section 1.14 with particular reference to 886 0.G. 638). The assignee of the present application has agreed that if the cell lines on deposit should die or be lost or destroyed when cultivated under suitable conditions, they will be promptly replaced on notification with a viable culture of the same cell line.

The present invention is not to be limited in scope by the microorganisms deposited, since the deposited embodiment is intended as a single illustration of one aspect of the invention and any microorganisms which are functionally equivalent are within the scope of this invention.

It is also to be understood that all base pair sizes given for nucleotides are approximate and are used for purpose of description.

The foreign gene is preferably coated onto carrier particles accelerated into the pollen.

The foreign gene may include a coding sequence and appropriate flanking regulatory sequences so that the coding sequence is expressed in tissues of the plant.

## Claims

1. A method of making genetically transformed living cells comprising the steps of: preparing copies of a foreign gene including a coding region and flanking regulatory sequences effective to express the coding region in the cells; coating copies of the foreign gene onto biologically inert carrier particles; layering the particles on a planar carrier sheet; accelerating the carrier sheet by impacting the carrier sheet with a shock wave, and restraining the carrier sheet such that the momentum of the carrier particles carries them from the sheet, so that some particles lodge in the interior of some of the cells; and screening for transformed cells.

2. A method according to claim 1 in which the shock wave generated to impact the carrier sheet is generated by high-voltage electric discharge through a spark gap bridged by a water droplet.

3. A method according to claim 1 to 2 in which the biologically inert particles are metallic.

4. A method according to claim 3 in which the metallic particles are gold spheres.

5. A method according to claim 3 or claim 4 which comprises the step of coating the metallic particles with an encapsulating agent before the gene copies are coated onto them.

6. A method according to claim 5 in which the encapsulating agent is polylysine.

7. A method according to either claim 5 or claim 6 in which the step of coating the foreign gene copies onto the metallic particles includes drying a solution of gene copies onto the particles.

8. A method according to claim 7 in which a solution of the gene copies include CaHPO₄ which is precipitated with the gene copies onto the metallic particles.

9. A method according to any one of the preceding claims in which the carrier particles are applied to the carrier sheet by suspending the carrier particles in ethanol, placing the ethanolic suspension on the carrier sheet and drying.

10. A method according to any one of the preceding claims in which the living cells are plant cells.

11. A method according to claim 10 in which the plant cells into which the carrier particles are accelerated are pollen cells; the pollen so treated being used to pollinate female organs of the plant and the progeny from the pollination screened for transformed progeny.

12. A method according to claim 11 in which a monolayer of plant pollen is placed on a target surface and the target surface placed in the acceleration path of the carrier particles.

13. A method according to claim 12 which comprises applying a layer of mineral oil to the target surface, dusting pollen thereon, and removing the excess oil therefrom.

14. A method according to either claim 12 or claim 13, in which the step of pollinating female organs of the plant is accomplished by brushing pollen into which particles have been accelerated off the target surface with a brush and dusting that pollen onto the female organs of a selected female parent plant.

15. A method according to any one of the preceding claims in which the screening for transformed cells is done on the basis of a biochemical assay.

16. A method according to claim 15 in which the assay is for antibiotic resistance.

17. A method according to any one of claims 10 to 14 in which the screening for transformed cells is done on the basis of a morphological plant trait.

18. A method according to any one of claims 10 to 17 in which the plants are maize.

19. Maize pollen comprising in it a foreign chimeric gene and at least one carrier particle which physically carried the foreign gene into the pollen.

20. Maize pollen according to claim 19 in which the foreign gene includes a coding sequence and appropriate flanking regulatory sequences so that the coding sequence is expressed in progeny of the pollen.

21. Apparatus for injecting carrier particles carrying DNA into living cells on a target, the apparatus comprising:
a planar carrier sheet arranged to receive on the surface of the sheet a layer of biologically inert carrier particles coated with DNA;
means for generating a shock wave sufficient to accelerate the carrier sheet with the carrier particles thereon to travel toward the target; and
restraining means disposed in the path of travel of the carrier sheet towards the target and arranged and constructed so as to restrain the carrier sheet when the carrier sheet is accelerated by a shock wave so that carrier particles are accelerated into cells on the target.

22. Apparatus according to claim 21 in which the means for generating a shock wave is constructed and arranged to generate a shock wave by high-voltage electric discharge through a spark gap bridged by a water droplet.

23. Apparatus according to claim 21 or 22 in which the means for generating a shock wave is adjustable so that the strength of the shock wave generated is adjustable.

24. Apparatus according to claim 21, 22 or 23 in which the restraining means comprises a retaining screen.

25. Apparatus for injecting carrier particles carrying DNA into living cells comprising: a spark discharge chamber (12); two electrodes (14) extending into the spark discharge chamber and spaced apart by a spark gap, the electrodes being adapted for attachment to an external source of high voltage discharge; a carrier sheet (18) held spaced above the spark discharge chamber, the carrier sheet receiving the carrier particles thereon; a retaining screen (20) fixed in place above the carrier sheet; and a target surface (22) held spaced above the retaining screen (20) and carrying the cells so that a spark discharge generating a shock wave in the discharge chamber (12) will accelerate the carrier sheet (18) into the retaining screen (20) so that the carrier particles are accelerated into the cells on the target surface (22).

26. Apparatus according to claim 25 in which a water droplet (24) is disposed bridging the gap between the electrodes (14).

27. Apparatus according to claim 25 or 26 which comprises a vacuum chamber to allow the apparatus to be operated in a partial vacuum.

28. Apparatus according to any one of claims 25 to 27 in which the spark gap between the electrodes is from 1 to 1.5 millimetres.

29. Apparatus according to any one of claims 25 to 28 in which a spacer ring (16) is positioned above the spark discharge chamber (12) but below the carrier sheet (18) and with at least a partial opening at its top.

30. Apparatus according to any one of claims 25 to 29 comprising an adjustable external source of high voltage discharge connected to the electrodes so that the high voltage discharge can be adjusted to a voltage of up to 15 kilovolts.

31. Apparatus according to claim 30 wherein the external source comprises a capacitor in which the high voltage discharge energy is stored until discharged through the electrodes.

32. Apparatus according to any one of claims 25 to 31 in which the carrier sheet is of aluminized mylar.

## Patentansprüche

1. Verfahren zur Herstellung genetisch transformierter lebender Zellen, umfassend die Schritte:
- Herstellung von Kopien eines Fremdgens einschließlich einer kodierenden Region und flankierender regulatorischer Sequenzen, die zur Expression der kodierenden Region in den Zellen wirksam sind,
- Beschichtung biologisch inerter Trägerpartikel mit Kopien des Fremdgens,
- Aufbringung der Partikel auf eine ebene Trägerfolie,
- Beschleunigung der Trägerfolie durch Einwirkung einer Verdichtungswelle auf die Trägerfolie, und Dämpfung der Trägerfolie in der Weise, daß die Trägerpartikel durch ihre Bewegungsenergie von der Folie fortgetragen werden, so daß sich einige Partikel in dem Inneren einiger Zellen ansammeln, und
- Screening nach transformierten Zellen.

2. Verfahren nach Anspruch 1, bei dem die zum Beschuß der Trägerfolie erzeugte Verdichtungswelle erzeugt wird durch eine elektrische Hochspannungsentladung über einen Elektrodenabstand, der durch einen Wassertropfen überbrückt ist.

3. Verfahren nach den Ansprüchen 1 und 2, bei dem die biologisch inerten Partikel metallisch sind.

4. Verfahren nach Anspruch 3, bei dem die metallischen Partikel Goldkügelchen sind.

5. Verfahren nach Anspruch 3 oder 4, umfassend den Schritt der Beschichtung der metallischen Partikel mit einem Einbettmittel, bevor sie mit den Genkopien beschichtet werden.

6. Verfahren nach Anspruch 5, bei dem das Einbettmittel Polylysin ist.

7. Verfahren nach einem der Ansprüche 5 oder 6, bei dem der Schritt der Beschichtung der metallischen Partikel mit den Fremdgenkopien das Trocknen einer Lösung von Genkopien auf den Partikeln einschließt.

8. Verfahren nach Anspruch 7, bei dem eine Lösung der Genkopien CaHPO₄ einschließt, welches mit den Genkopien auf die metallischen Partikel präzipitiert wird.

9. Verfahren nach einem der vorhergehenden Ansprüche, bei dem die Trägerpartikel auf die Trägerfolie aufgebracht werden, indem die Trägerpartikel in Ethanol suspendiert werden und die ethanolische Suspension auf die Trägerfolie aufgebracht und getrocknet wird.

10. Verfahren nach einem der vorhergehenden Ansprüche, bei dem die lebenden Zellen Pflanzenzellen sind.

11. Verfahren nach Anspruch 10, bei dem die Pflanzenzellen, in die die Trägerpartikel durch Beschleunigung eintreten, Pollenzellen sind, wobei die auf diese Weise behandelten Pollen zur Bestäubung weiblicher Organe der Pflanze verwendet werden und die Nachkommenschaft aus der Bestäubung hinsichtlich transformierter Nachkommen abgesucht wird.

12. Verfahren nach Anspruch 11, bei dem eine Monoschicht von Pflanzenpollen auf eine Zieloberfläche aufgebracht wird, und die Zieloberfläche in den Beschleunigungsweg der Trägerpartikel plaziert wird.

13. Verfahren nach Anspruch 12, umfassend das Aufbringen einer Schicht aus Mineralöl auf die Zieloberfläche, das Aufstäuben von Pollen darauf, und das Entfernen von überschüssigem Öl davon.

14. Verfahren nach einem der Ansprüche 12 oder 13, bei dem der Schitt der Bestäubung weiblicher Organe der Pflanze erfolgt, indem Pollen, in die Partikel mittels Beschleunigung eingedrungen sind, von der Zieloberfläche mit einem Pinsel abgebürstet werden und diese Pollen auf die weiblichen Organe einer ausgewählten weiblichen Mutterpflanze aufgestäubt werden.

15. Verfahren nach einem der vorhergehenden Ansprüche, bei dem das Screening nach transformierten Zellen auf der Grundlage eines biochemischen Assays erfolgt.

16. Verfahren nach Anspruch 15, bei dem sich der Assay auf Antibiotika-Resistenz bezieht.

17. Verfahren nach einem der Ansprüche 10 bis 14, bei dem das Screening nach transformierten Zellen auf der Grundlage einer morphologischen Pflanzeneigenschaft erfolgt.

18. Verfahren nach einem der Ansprüche 10 bis 17, bei dem die Pflanzen Mais sind.

19. Maispollen, der ein fremdes chimäres Gen und mindestens ein Trägerpartikel umfaßt, welches das Fremdgen physikalisch in den Pollen eingeführt hat.

20. Maispollen nach Anspruch 19, in dem das Fremdgen eine kodierende Sequenz und geeignete flankierende regulatorische Sequenzen einschließt, so daß die kodierende Sequenz in den Nachkommen des Pollens exprimiert wird.

21. Vorrichtung zur Injektion von DNA-tragenden Trägerpartikeln in lebende Zellen auf einer Zieloberfläche, wobei die Vorrichtung umfaßt:
- eine ebene Trägerfolie, die so eingerichtet ist, daß auf der Oberfläche der Folie eine Schicht von mit DNA beschichteten biologisch inerten Trägerpartikeln aufgebracht werden kann,
- Mittel zur Erzeugung einer ausreichenden Verdichtungswelle, um die Trägerfolie mit den darauf befindlichen Trägerpartikeln in Richtung der Zieloberfläche zu beschleunigen, und
- Dämpfungsmittel, das sich in der Fortbewegungsrichtung der Trägerfolie zur Zieloberfläche befindet und derart angeordnet und konstruiert ist, daß die Trägerfolie gedämpft wird, wenn die Trägerfolie durch eine Verdichtungswelle beschleunigt wird, so daß die Trägerpartikel durch die Beschleunigung in die Zellen auf der Zieloberfläche eindringen.

22. Vorrichtung nach Anspruch 21, bei der das Mittel zur Erzeugung einer Verdichtungswelle in der Weise konstruiert und angeordnet ist, daß eine Verdichtungswelle erzeugt wird durch eine elektrische Hochspannungsentladung über einen Elektrodenabstand, der durch einen Wassertropfen überbrückt ist.

23. Vorrichtung nach Anspruch 21 oder 22, bei der das Mittel zur Erzeugung einer Verdichtungswelle einstellbar ist, so daß die Stärke der erzeugten Verdichtungswelle einstellbar ist.

24. Vorrichtung nach den Ansprüchen 21, 22 oder 23, bei der das Dämpfungsmittel ein Rückhaltesieb umfaßt.

25. Vorrichtung zum Injizieren von DNA-tragenden Partikeln in lebende Zellen, umfassend: eine Funkenentladungskammer (12); zwei Elektroden (14), die in die Funkenentladungskammer hineinragen und durch einen Elektrodenabstand voneinander entfernt sind, wobei die Elektroden derart beschaffen sind, daß sie an eine externe Hochspannungsentladungsquelle angeschlossen werden können; eine Trägerfolie (18), die in einem Abstand oberhalb der Funkenentladungskammer angeordnet ist und die Trägerpartikel empfängt; ein Rückhaltesieb (20), das oberhalb der Trägerfolie angeordnet ist; und eine Zieloberfläche (22), die in einem Abstand oberhalb des Rückhaltesiebs (20) angeordnet ist und die Zellen trägt, so daß die Trägerfolie (18) durch die in der Entladungskammer (12) mittels Funkenentladung erzeugte Verdichtungswelle in Richtung des Rückhaltesiebs (20) beschleunigt wird, so daß die Trägerpartikel mittels Beschleunigung in die Zellen auf der Zieloberfläche (22) eindringen.

26. Vorrichtung nach Anspruch 25, bei der zur Überbrückung des Abstandes zwischen den Elektroden (14) ein Wassertropfen (24) vorgesehen ist.

27. Vorrichtung nach Anspruch 25 oder 26, die eine Vakuumkammer umfaßt, um den Betrieb der Vorrichtung in einem Teilvakuum zu ermöglichen.

28. Vorrichtung nach einem der Ansprüche 25 bis 27, bei der der Elektrodenabstand zwischen den Elektroden 1 bis 1,5 mm beträgt.

29. Vorrichtung nach einem der Ansprüche 25 bis 28, bei der ein Distanzring (16) oberhalb der Funkenentladungskammer (12), aber unterhalb der Trägerfolie (18) angeordnet ist und an seinem oberen Ende mindestens eine partielle Öffnung aufweist.

30. Vorrichtung nach einem der Ansprüche 25 bis 29, umfassend eine einstellbare, externe Quelle für eine Hochspannungsentladung, die derart an die Elektroden angeschlossen ist, daß die Hochspannungsentladung auf eine Spannung von bis zu 15 kV eingestellt werden kann.

31. Vorrichtung nach Anspruch 30, bei der die externe Quelle einen Kondensator umfaßt, in dem die Hochspannungs-Entladungsenergie bis zur Entladung durch die Elektroden gespeichert wird.

32. Vorrichtung nach einem der Ansprüche 25 bis 31, in der die Trägerfolie aus aluminisiertem Mylar ist.

## Revendications

1. Un procédé d'élaboration de cellules vivantes génétiquement transformées, comprenant les opérations consistant : à préparer des copies d'un gêne étranger comportant une région de codage et des séquences régulatoires flanquantes efficaces pour exprimer la région de codage dans les cellules; à revêtir des particules porteuses biologiquement inertes de copies du gêne étranger; à disposer les particules en couche sur une feuille porteuse plane; à accélérer la feuille porteuse en frappant la feuille porteuse par une onde de choc et à retenir la feuille porteuse, de façon que la force vive des particules porteuses les transporte depuis la feuille, de sorte que certaines particules se logent à l'intérieur de quelques unes des cellules; et à déceler, par filtrage, des cellules transformées.

2. Un procédé selon la revendication 1, dans lequel l'onde de choc, engendrée pour frapper la feuille porteuse, est engendrée par une décharge électrique à haute tension à travers un intervalle d'éclatement ponté par une gouttelette d'eau.

3. Un procédé selon la revendication 1 ou 2, dans lequel les particules biologiquement inertes sont métalliques.

4. Un procédé selon la revendication 3, dans lequel les particules métalliques sont des sphères d'or.

5. Un procédé selon la revendication 3 ou la revendication 4, qui comprend l'opération de revêtement des particules métalliques par un agent encapsulant avant que leur soient appliquées les copies de gêne.

6. Un procédé selon la revendication 5, dans lequel l'agent encapsulant est de la polylysine.

7. Un procédé selon la revendication 5 ou la revendication 6, dans lequel l'opération d'application des copies de gêne étranger sur les particules métalliques comprend le séchage d'une solution de copies de gêne sur les particules.

8. Un procédé selon la revendication 7, dans lequel une solution des copies de gêne comprend du CaHPO₄ qui est précipité avec les copies de gêne sur les particules métalliques.

9. Un procédé selon l'une quelconque des revendications précédentes, dans lequel les particules porteuses sont appliquées à la feuille porteuse en suspendant les particules porteuses dans de l'éthanol, en plaçant la suspension éthalonique sur la feuille porteuse et en séchant.

10. Un procédé selon l'une quelconque des revendications précédentes, dans lequel les cellules vivantes sont des cellules végétales.

11. Un procédé selon la revendication 10, dans lequel les cellules végétales, dans lesquelles se logent les particules porteuses après avoir été accélérées, sont des cellules de pollen; le pollen ainsi traité étant utilisé pour polliniser des organes femelles de la plante et les descendants de la pollinisation étant filtrés pour déceler les descendants transformés.

12. Un procédé selon la revendication 11, dans lequel une monocouche de pollen végétal est placée sur une surface-cible et la surface-cible placée dans le trajet d'accélération des particules porteuses.

13. Un procédé selon la revendication 12, qui comprend les opérations consistant à appliquer une couche d'huile minérale sur la surface-cible, à atomiser du pollen sur celle-ci et à éliminer l'huile en excès de celle-ci.

14. Un procédé selon la revendication 12 ou la revendication 13, dans lequel l'opération de pollinisation d'organes femelles de la plante est accomplie en éliminant, de la surface-cible par brossage avec une brosse, le pollen dans lequel se sont logées des particules qui ont été accélérées et en pulvérisant ce pollen sur les organes femelles d'une plante femelle parente sélectionnée.

15. Un procédé selon l'une quelconque des revendications précédentes, dans lequel le filtrage de détection de cellules transformées est fait sur la base d'un essai biochimique.

16. Un procédé selon la revendication 15, dans lequel l'essai est effectué pour la résistance antibiotique.

17. Un procédé selon l'une quelconque des revendications 10 à 14, dans lequel le filtrage de détection de cellules transformées est fait sur la base d'un trait morphologique de la plante.

18. Un procédé selon l'une quelconque des revendications 10 à 17, dans lequel les plantes sont du maïs.

19. Pollen de maïs comprenant en soi un gêne chimérique étranger et au moins une particule porteuse qui transporte physiquement le gêne étranger pour l'introduire dans le pollen.

20. Pollen de maïs selon la revendication 19, dans lequel le gêne étranger comprend une séquence de codage et des séquences régulatoires flanquantes appropriées, de façon que la séquence de codage soit exprimée dans les descendants du pollen.

21. Appareil pour injecter des particules porteuses amenant du ADN dans des cellules vivantes sur une cible, l'appareil comprenant :
une feuille porteuse plane agencée pour recevoir, sur la surface de la feuille, une couche de particules porteuses biologiquement inertes revêtues de ADN;
des moyens pour engendrer une onde de choc suffisante pour accélérer la feuille porteuse avec les particules porteuses sur celle-ci, pour cheminer en direction de la cible; et
des moyens de retenue disposés dans la trajectoire de mouvement de la feuille porteuse en direction de la cible et agencés et construits de façon à retenir la feuille porteuse lorsque la feuille porteuse est accélérée par une onde de choc, de façon que des particules porteuses sont accélérées pour se loger dans les cellules sur la cible.

22. Appareil selon la revendication 21, dans lequel le moyen pour engendrer une onde de choc est construit et agencé pour engendrer une onde de choc par une décharge électrique à haute tension à travers un intervalle d'éclatement ponté par une gouttelette d'eau.

23. Appareil selon la revendication 21 ou 22, dans lequel le moyen pour engendrer une onde de choc est réglable, de façon que l'intensité de l'onde de choc engendrée soit réglable.

24. Appareil selon la revendication 21, 22 ou 23, dans lequel le moyen de retenue comprend un écran de retenue.

25. Appareil pour injecter des particules porteuses transportant du ADN pour les loger dans des cellules vivantes, comprenant : une chambre de décharge à étincelles (12); deux électrodes (14) pénétrant dans la chambre de décharge à étincelle et espacées d'un intervalle d'éclatement, les électrodes étant destinées à la connexion à une source externe de décharge à haute tension; une feuille porteuse (18) maintenue espacée au-dessus de la chambre de décharge à étincelle, la feuille porteuse recevant les particules porteuses sur celle-ci; un écran de retenue (20) fixé en place au-dessus de la feuille porteuse; et une surface-cible (22) maintenue espacée au-dessus de l'écran de retenue (20) et portant les cellules, de sorte qu'une décharge à étincelle, engendrant une onde de choc dans la chambre de décharge (12), accélérera la feuille porteuse (18) pour l'amener dans l'écran de retenue (20), de sorte que les particules porteuses sont accélérées pour se loger dans les cellules sur la surface-cible (22).

26. Appareil selon la revendication 25, dans lequel une gouttelette d'eau (24) est disposée de façon à ponter l'intervalle entre les électrodes (14).

27. Appareil selon la revendication 25 ou 26, qui comprend une chambre à vide pour permettre de faire fonctionner l'appareil dans un vide partiel.

28. Appareil selon l'une quelconque des revendications 25 à 27, dans lequel l'intervalle d'éclatement entre les électrodes est de 1 à 1,5 millimètre.

29. Appareil selon l'une quelconque des revendications 25 à 28, dans lequel une bague entretoise (16) est positionnée au-dessus de la chambre de décharge à étincelle (12) mais en dessous de la feuille porteuse (18) et à au moins une ouverture partielle à son sommet.

30. Appareil selon l'une quelconque des revendications 25 à 29, comprenant une source externe réglable de décharge à haute tension connectée aux électrodes, de façon que la décharge à haute tension puisse être ajustée à une tension allant jusqu'à 15 kilovolts.

31. Appareil selon la revendication 30, dans lequel la source externe comprend un condensateur dans lequel l'énergie de décharge à haute tension est emmaganisée jusqu'à ce qu'elle soit déchargée entre les électrodes.

32. Appareil selon l'une quelconque des revendications 25 à 31, dans lequel la feuille porteuse est du Mylar aluminisé.
